# EUROPEAN PATENT APPLICATION

(11) **EP 4 169 452 A1**
(43) Date of publication of application: **26.04.2023**
(21) Application number: 22202237.8
(22) Date of filing: 18.10.2022
(51) Int. Cl.: A61B 8/12, A61B 8/00

(54) **ULTRASONIC DIAGNOSTIC APPARATUS, INFORMATION-PROCESSING METHOD, AND STORAGE MEDIUM**

(30) Priority: 20.10.2021 JP 2021171749
(71) Applicant: Canon Medical Systems Corporation, Tochigi (JP)
(72) Inventor: OGASAWARA, Yoichi, Tochigi (JP); MAKITA, Yasuhisa, Tochigi (JP); TAKEDA, Sakie, Tochigi (JP)
(74) Representative: Marks & Clerk LLP

(57) **Abstract**

An ultrasonic diagnostic apparatus of an embodiment includes an acquisition unit and an output control unit. The acquisition unit acquires contact information detected by a plurality of contact sensors provided at different positions on an ultrasonic probe inserted into a body cavity of a subject. The output control unit causes an output interface to output information representing a state of the ultrasonic probe in the body cavity on the basis of the contact information and the positions of the contact sensors provided in the ultrasonic probe.

## Description

### FIELD

Embodiments disclosed in the present description and drawings relate to an ultrasonic diagnostic apparatus, an information-processing method, and a storage medium.

### BACKGROUND

To obtain information on a cross section, three-dimensional tissue structure, or physical properties of a diagnostic part of a living body, an ultrasonic diagnostic apparatus that images information in the living body by transmitting ultrasonic beams, receiving reflected waves from tissue boundaries within a subject having different acoustic impedances, and processing the received signal has been commercialized.

There are various types of ultrasonic probes serving as sensors for transmitting and receiving ultrasonic beams depending on diagnostic parts, such as ones applied to the surface of the body and ones that are inserted into body cavities. For example, there is a transesophageal echocardiography (TEE) probe having an ultrasonic transducer at the tip or the like in order to observe the state of the heart of a subject through the esophagus. This transesophageal echocardiography probe is used by being inserted into upper digestive organs of a subject, such as the esophagus and stomach. An ultrasonic diagnostic apparatus can acquire and display the state of the heart and the like using the transesophageal echocardiography probe inserted into the upper digestive organs.

For example, there is a transesophageal echocardiography probe having an array-shaped ultrasonic transducer disposed at the tip thereof and capable of manually rotating around the center of the ultrasonic transducer or automatically rotating by a motor or the like. This transesophageal echocardiography probe may further include a container filled with an ultrasound transmission medium.

The upper digestive organs such as the esophagus and stomach differ in size and shape depending on the living body. Therefore, it is required to efficiently insert the transesophageal echocardiography probe into the digestive organs or to bring the ultrasonic transducer surface into close contact with the inner wall surface with an appropriate pressure for the purpose of obtaining optimum in-vivo information, and the structure near the tip of the probe is bendable and needs to have flexibility for bending.

On the other hand, due to this flexibility, the transesophageal echocardiography probe may be bent when inserted into the esophagus which has various shapes depending on the living body. Since the transesophageal echocardiography probe receives reflected ultrasonic waves to generate an ultrasonic image, the ultrasonic image may not be displayed while it is inserted into the esophagus. That is, even if the transesophageal echocardiography probe is bent in the esophagus, it is difficult for an operator to check this state. Despite the fact that the transesophageal echocardiography probe is bent in the esophagus in this manner, the operator cannot check the state of the transesophageal echocardiography probe, and thus there is a risk of damaging the inner wall of the esophagus by the tip of the transesophageal echocardiography probe being hooked on the esophagus when the operator intends to remove the transesophageal echocardiography probe in a bent state.

In addition, bending degree control for bringing the ultrasonic transducer surface into close contact with the inner wall surface with an appropriate pressure is performed according to an operator's experience depending on an image displayed on an ultrasonic diagnostic apparatus and a subtle sense of resistance of the transesophageal echocardiography probe at hand, and thus an objective and appropriate amount of bending cannot be ascertained, which causes a burden on the inner wall due to the probe being pressed against the inner wall with more pressure than necessary, or an ultrasonic image with a sufficient signal-to-noise (S/N) ratio cannot be acquired due to pressing with insufficient bending, which may reduce diagnostic efficiency.

Furthermore, the above-mentioned problems such as the risk of damage to the body cavity and a decrease in diagnostic efficiency are not limited to the transesophageal echocardiography probe and are generally common to ultrasonic probes inserted into the body cavity, such as transvaginal ultrasonic probes and transrectal ultrasonic probes.

### SUMMARY

The task to be solved by embodiments disclosed in the present description and drawings is to improve safety and diagnostic efficiency. However, the task to be solved by the embodiments disclosed in the present description and the drawings is not limited to the aforementioned task. A task corresponding to each effect according to each configuration shown in an embodiment which will be described later can be positioned as another task.

An ultrasonic diagnostic apparatus according to an embodiment includes an acquisition unit and an output control unit. The acquisition unit acquires contact information detected by a plurality of contact sensors provided at different positions on an ultrasonic probe inserted into a body cavity of a subject. The output control unit causes an output interface to output information representing a state of the ultrasonic probe in the body cavity on the basis of the contact information and the positions of the contact sensors provided in the ultrasonic probe. In an embodiment, the state of the ultrasonic probe indicates whether the probe is bent or not.

### BRIEF DESCRIPTION OF THE DRAWINGS

FIG. 1 is a configuration diagram of an ultrasonic diagnostic apparatus of a first embodiment.
FIG. 2 is an external view of an ultrasonic probe of the first embodiment.
FIG. 3 is an enlarged view of an insertion part of the ultrasonic probe of the first embodiment.
FIG. 4 is a flowchart showing a flow of a series of processing of a processing circuitry of the first embodiment.
FIG. 5 is a diagram showing an example of a bull's eye map.
FIG. 6 is a diagram schematically showing a state in which the insertion part has been inserted into a body cavity.
FIG. 7 is a diagram showing an example of a bull's eye map in the case of Fig. 6.
FIG. 8 is a diagram schematically showing a state in which the insertion part has been inserted into a body cavity.
FIG. 9 is a diagram showing an example of a bull's eye map in the case of Fig. 8.
FIG. 10 is a diagram schematically showing a state in which the insertion part has been inserted into a body cavity.
FIG. 11 is a diagram showing an example of a bull's eye map in the case of Fig. 10.
FIG. 12 is a diagram describing a method of estimating a degree of risk using a pressure value and a bending degree.
FIG. 13 is a diagram describing a method of estimating a degree of risk using machine learning.
FIG. 14 is a diagram showing an example of a display screen.
FIG. 15 is a diagram showing an example of a display screen.
FIG. 16 is a diagram showing an example of a display screen.
FIG. 17 is a diagram showing an example of a display screen.
FIG. 18 is a flowchart showing a flow of a series of processing of a processing circuitry of a second embodiment.

### DETAILED DESCRIPTION

An ultrasonic diagnostic apparatus, an information-processing method, and a storage medium according to embodiments will be described below with reference to the drawings.

### (First embodiment)

### [Configuration of ultrasonic diagnostic apparatus]

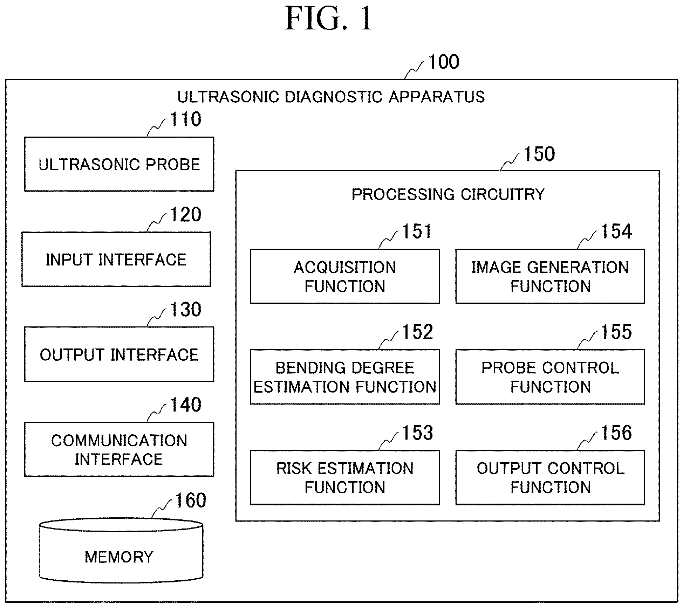

Fig. 1 is a configuration diagram of an ultrasonic diagnostic apparatus 100 according to a first embodiment. As illustrated, the ultrasonic diagnostic apparatus 100 includes an ultrasonic probe 110, an input interface 120, an output interface 130, a communication interface 140, a processing circuitry 150, and a memory 160.

The ultrasonic probe 110 is operated by a user such as a doctor or a nurse and generally includes a type that contacts the body surface of a subject and a type that is inserted into the body cavity. In the present embodiment, a probe of the type that is inserted into the body cavity will be described below. The ultrasonic probe 110 may be a transesophageal echocardiography probe, or a transvaginal or transrectal ultrasonic probe. For example, if the ultrasonic probe 110 is a transesophageal echocardiography probe, the ultrasonic probe 110 is inserted into upper digestive organs such as the esophagus and stomach. A subject is, for example, a human being, but is not limited thereto. The ultrasonic probe 110 transmits (radiates) ultrasonic waves to a subject, for example, in order to acquire an image inside the body cavity. The ultrasonic probe 110 receives echoes (reflected waves) of the transmitted ultrasonic waves. The ultrasonic probe 110 then generates signal data of the received echo (hereinafter referred to as echo data) and outputs the echo data to the ultrasonic diagnostic apparatus 100. For example, the ultrasonic probe 110 may be a probe in which a plurality of transducers for transmitting and receiving ultrasonic waves are one-dimensionally arranged and have a rotating mechanism, or a two-dimensional array probe in which a plurality of transducers are arranged in a two-dimensional array. In this case, the echo data may be, for example, three-dimensional volume data in which an echo signal intensity is associated with each unit area obtained by dividing a three-dimensional space having a width, a height, and a depth.

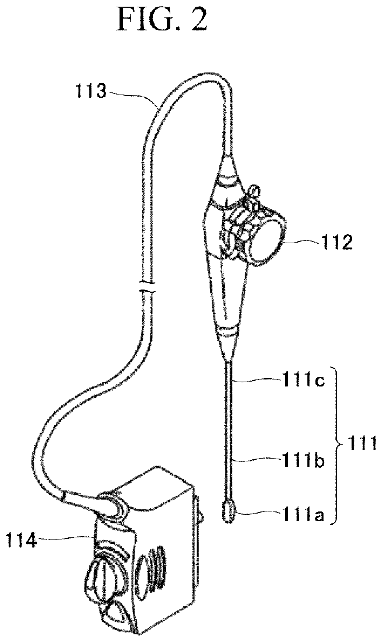

Fig. 2 is an external view of the ultrasonic probe 110 of the first embodiment. As illustrated, the ultrasonic probe 110 includes an insertion part 111, an operation part 112, a probe cable 113, and a connector part 114.

The insertion part 111 is a part that is inserted into the body cavity and has a tip portion 111a, a bending portion 111b, and a guide intermediate portion 111c. A transducer T for transmitting and receiving ultrasonic waves is attached to the tip portion 111a. The bending portion 111b is a portion of the insertion part 111 which has a bending structure. Due to the presence of the bending portion 111b, the insertion part 111 can be easily inserted into the body cavity, the transducer T can be brought into close contact with the inner wall surface of the body cavity with an appropriate pressure, or an ultrasonic beam can be transmitted to an organ that is a diagnosis target (for example, the heart) with high efficiency. The guide intermediate portion 111c is an intermediate portion of the insertion part 111 which does not have a bending structure. In a bending direction of the bending portion 111b, since the transducer T of the tip portion 111a is pressed against the inner wall in general, a side where the bending portion 111b bends and shrinks is referred to as a front side and a side where the bending portion 111b warps and stretches conversely is referred to as a back side for convenience of description.

A transducer rotation knob, a vertical bending knob, a lock lever, and the like which are can be operated by a user are attached to the operation part 112. The transducer rotation knob is an operator for rotating the insertion part 111 in the extending direction thereof. The vertical bending knob is an operator for bending the bending portion 111b toward the front side or stretching it toward the back side. The lock lever is an operator for fixing the insertion part 111 moved by operating the transducer rotation knob or the vertical bending knob at that position.

The operation part 112 includes an actuator (for example, an electric motor or the like) that rotates, bends, or stretches the insertion part 111 without depending on a user operation performed on operators such as the transducer rotation knob and the vertical bending knob.

The probe cable 113 is a cable that electrically connects the insertion part 111 and the operation part 112 with the connector part 114.

The connector part 114 is an input/output interface electrically connected to the ultrasonic diagnostic apparatus 100 or a terminal thereof.

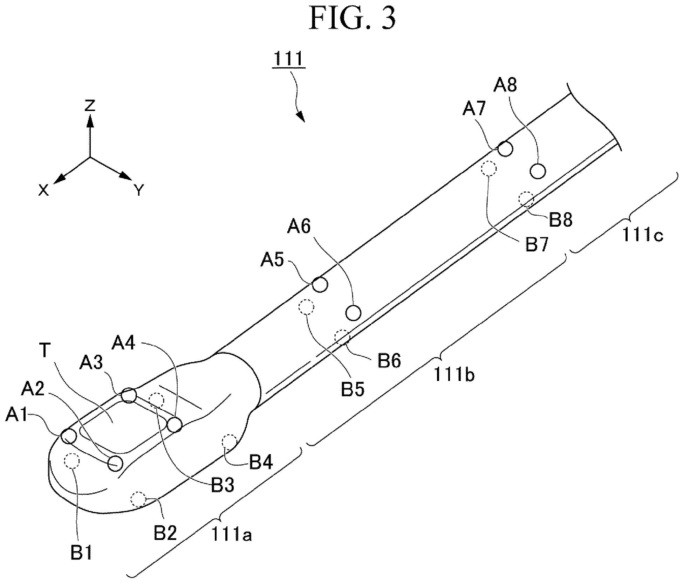

Fig. 3 is an enlarged view of the insertion part 111 of the ultrasonic probe 110 of the first embodiment. When the ultrasonic probe 110 is inserted into the body cavity, it is difficult to actually check how the insertion part 111 is in contact with the body cavity. Therefore, a plurality of pressure sensors A and B are attached to the insertion part 111 in order to sense the state of the insertion part 111 in the body cavity.

As shown in Fig. 3, for example, pressure sensors A1 to A8 are provided on the front surface (surface on the side of a positive Z direction) of the insertion part 111, and pressure sensors B1 to B8 are provided on the back surface (surface on the side of a negative Z direction) of the insertion part 111 symmetrically with the pressure sensors A1 to A8. In the extending direction (X direction) of the ultrasonic probe 110, (A1, A3, A5, A7) and (A2, A4, A6, A8) are in a symmetrical positional relationship and (B1, B3, B5, B7) and (B2, B4, B6, B8) are in a symmetrical positional relationship. Further, in the front-and-back direction (Z direction) of the ultrasonic probe 110, (A1, A3, A5, A7) and (B1, B3, B5, B7) are in a symmetrical positional relationship, and (A2, A4, A6, A8) and (B2, B4, B6, B8) are in a symmetrical positional relationship. When each pressure sensor detects pressure, it outputs information or a signal (hereinafter referred to as pressure information) representing the intensity of the detected pressure to the ultrasonic diagnostic apparatus 100 via the probe cable 113 and connector part 114. Note that the positional relationships of these pressure sensors do not need to be completely symmetrical in the extending direction (X direction) and/or the front-and-back direction (X direction) and may be partially asymmetrical. Moreover, the pressure sensors A and B are not limited to those capable of detecting local pressure applied thereto and may be of a thin film type capable of detecting a wide range of pressure. Furthermore, the pressure information may include information representing whether or not a pressure sensor comes into contact with the inner wall of the body cavity (for example, a binary value indicating 1 if a pressure sensor is in contact and 0 if not in contact). The pressure sensor is an example of a "contact sensor," a pressure detected by a pressure sensor is an example of a "contact force," and pressure information is an example of "contact information."

A description of the configuration diagram of the ultrasonic diagnostic apparatus 100 in Fig. 1 will now continue. The input interface 120 receives various input operations from the user, converts the received input operations into electrical signals, and outputs the electrical signals to the processing circuitry 150. The input interface 120 includes physical operation components such as a mouse, keyboard, trackball, switch, button, joystick, and touch panel. The input interface 120 may be, for example, a user interface that receives audio input, such as a microphone. When the input interface 120 is a touch panel, the input interface 120 may also have a display function of a display 130a which will be described later.

In the present description, the input interface 120 is not limited to one including physical operation components such as a mouse and a keyboard. For example, examples of the input interface 120 also include an electrical signal processing circuitry that receives an electrical signal corresponding to an input operation from an external input device provided separately from the apparatus and outputs the electrical signal to a control circuit.

The output interface 130 includes, for example, the display 130a and a speaker 130b. The display 130a displays various types of information. For example, the display 130a displays information output by the processing circuitry 150 as an image or displays a graphical user interface (GUI) for receiving various input operations from the user. For example, the display 130a is a liquid crystal display (LCD), an organic electroluminescence (EL) display, or the like. The speaker 130b outputs information output by the processing circuitry 150 as sound. The output interface 130 is an example of an "output unit."

The communication interface 140 includes, for example, a network interface card (NIC) and an antenna for wireless communication. The communication interface 140 communicates with the ultrasonic probe 110 connected via the connector part 114 and communicates with an external device via a communication network NW.

The communication network NW may be any information communication network using telecommunication technology. For example, the communication network NW includes a local area network (LAN), a wide area network (WAN), the Internet, a telephone communication network, an optical fiber communication network, a cable communication network, a satellite communication network, or the like.

The external device may be, for example, a computer connected to a hospital backbone LAN. That is, the external device may be a computer in a medical institution where the ultrasonic diagnostic apparatus 100 is installed. Such a computer is also called a workstation. Further, the external device may be a cloud server or the like connected to a WAN or the Internet.

When the ultrasonic probe 110 is a wireless probe, the communication interface 140 may communicate with the ultrasonic probe 110 wirelessly such as through Wi-Fi.

The processing circuitry 150 includes, for example, an acquisition function 151, a bending degree estimation function 152, a risk estimation function 153, an image generation function 154, a probe control function 155, and an output control function 156. The processing circuitry 150 realizes these functions by a hardware processor (computer) executing a program stored in the memory 160 (storage circuit), for example. The acquisition function 151 is an example of an "acquisition unit" and a combination of the bending degree estimation function 152 and the risk estimation function 153 is an example of an "estimation unit," a "generation unit," or a "determination unit." The probe control function 155 is an example of a "probe control unit" and the output control function 156 is an example of an "output control unit."

The hardware processor in the processing circuitry 150 refers to, for example, a circuit (circuitry) such as a central processing unit (CPU), a graphics processing unit (GPU), an application specific integrated circuit (ASIC), or a programmable logic device (for example, a simple programmable logic device (SPLD), a complex programmable logic device (CPLD), or a field programmable gate array (FPGA)). Instead of the program being stored in the memory 160, the program may be directly incorporated into the circuit of the hardware processor. In this case, the hardware processor realizes functions by reading and executing the program incorporated in the circuit. The aforementioned program may be stored in the memory 160 in advance, or may be stored in a non-transitory storage medium such as a DVD or a CD-ROM and installed in the memory 160 from the non-transitory storage medium by setting the non-transitory storage medium in a drive device (not shown) of the ultrasonic diagnostic apparatus 100. The hardware processor is not limited to being configured as a single circuit and may be configured as one hardware processor by combining a plurality of independent circuits to realize each function. Further, a plurality of components may be integrated into one hardware processor to realize each function.

For example, the acquisition function 151 acquires echo data from the ultrasonic probe 110 and acquires pressure information from each pressure sensor provided on the ultrasonic probe 110 via the communication interface 140.

The bending degree estimation function 152 estimates a bending state of the ultrasonic probe 110 on the basis of the pressure information acquired from each pressure sensor by the acquisition function 151 and the installation position of each pressure sensor.

The risk estimation function 153 estimates a degree of risk that the ultrasonic probe 110 will damage the inner wall of the body cavity on the basis of at least the pressure information between the pressure information acquired from each pressure sensor by the acquisition function 151 and the bending state of the ultrasonic probe 110 estimated by the bending degree estimation function 152, or both the pressure information and the bending state.

Further, the risk estimation function 153 may estimate or determine whether or not the surface of the transducer T (ultrasonic radiation surface) is in contact with the inner wall of the body cavity with a predetermined pressure on the basis of the pressure information.

The image generation function 154 generates an ultrasonic image of the inside of the body cavity on the basis of the echo data acquired by the acquisition function 151.

The probe control function 155 controls the bending degree of the ultrasonic probe 110 on the basis of the bending state of the ultrasonic probe 110 estimated by the bending degree estimation function 152.

The output control function 156 outputs the pressure information acquired from each pressure sensor by the acquisition function 151 via the output interface 130. The output control function 156 may also output the bending state estimated by the bending degree estimation function 152 via the output interface 130. In addition, the output control function 156 may output the degree of risk estimated by the risk estimation function 153 or a result of determination of whether or not the transducer T is in contact with the inner wall of the body cavity with a predetermined pressure via the output interface 130. The bending state, the degree of risk, and the like are examples of "information based on a bending state."

The memory 160 is realized by, for example, a random access memory (RAM), a semiconductor memory device such as a flash memory, a hard disk, or an optical disc. These non-transitory storage media may be realized by other storage devices such as a network attached storage (NAS) and external storage server devices connected via the communication network NW. Further, the memory 160 may also include non-transitory storage media such as a read only memory (ROM) and registers.

### [Processing flow of ultrasonic diagnostic apparatus]

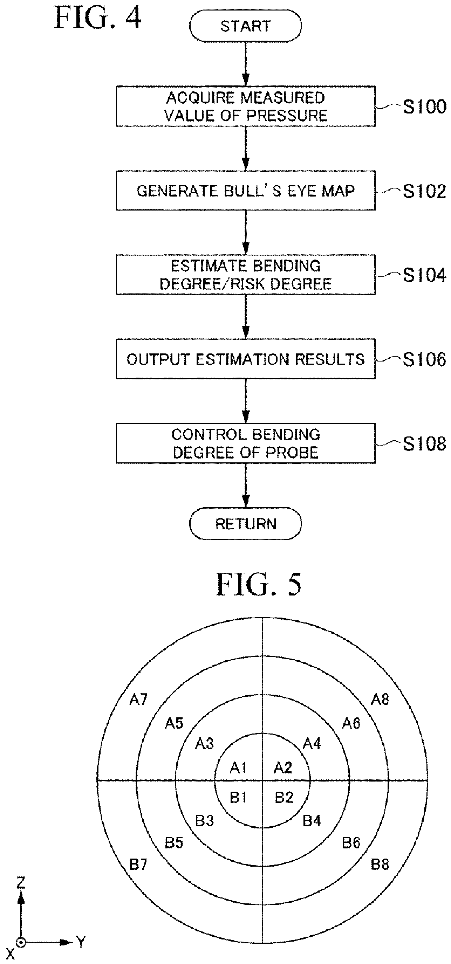

Processing of each function performed by the processing circuitry 150 of the ultrasonic diagnostic apparatus 100 according to the first embodiment will be described below with reference to a flowchart. Fig. 4 is a flowchart showing a series of processing of the processing circuitry 150 of the first embodiment. Processing of this flowchart may be repeatedly executed at a predetermined period, for example. The predetermined cycle may be, for example, at least once per second or more.

First, the acquisition function 151 acquires pressure information from each pressure sensor of the ultrasonic probe 110 in accordance with a predetermined cycle (step S100).

Next, the risk estimation function 153 generates a bull's eye map on the basis of the pressure information acquired from each pressure sensor by the acquisition function 151 and the installation position of each pressure sensor (step S102). A bull's eye map is a pressure distribution in which pressure values indicated by pressure information of respective pressure sensors are arranged and displayed on one concentric circle. A bull's eye map is an example of a "contact information map."

Fig. 5 is a diagram showing an example of a bull's eye map. It is assumed that the pressure sensors A1 to A8 are provided on the front surface of the insertion part 111 and the pressure sensors B1 to B8 are provided on the back surface of the insertion part 111 as described above with reference to Fig. 3. In this case, the pressure value of a pressure sensor closer to the tip of the insertion part 111 is disposed at the center.

Specifically, the pressure value of the pressure sensor A2, the pressure value of the pressure sensor A4, the pressure value of the pressure sensor A6, and the pressure value of the pressure sensor A8 are arranged and displayed in order from the center to the outside in the first quadrant of the bull's eye map. The pressure value of the pressure sensor A1, the pressure value of the pressure sensor A3, the pressure value of the pressure sensor A5, and the pressure value of the pressure sensor A7 are arranged and displayed in order from the center to the outside in the second quadrant of the bull's eye map. The pressure value of the pressure sensor B1 the pressure value of the pressure sensor B3, the pressure value of the pressure sensor B5, and the pressure value of the pressure sensor B7 are arranged and displayed in order from the center to the outside in the third quadrant of the bull's eye map. The pressure value of the pressure sensor B2, the pressure value of the pressure sensor B4, the pressure value of the pressure sensor B6, and the pressure value of the pressure sensor B8 are arranged and displayed in order from the center to the outside in the fourth quadrant of the bull's eye map. The bull's eye map may be represented by a heat map in which each pressure value is replaced with a pixel value such as a grayscale, luminance, or saturation. The following description is based on the assumption that a dark-colored portion (dark portion, close to black) corresponds to a low pressure and a light-colored portion (bright portion, close to white) corresponds to a high pressure on a bull's eye map.

A description of the flowchart of Fig. 4 will now continue. Next, the bending degree estimation function 152 estimates the bending state of the ultrasonic probe 110 on the basis of the bull's eye map, and the risk estimation function 153 estimates a degree of risk that the ultrasonic probe 110 will damage the inner wall of the body cavity on the basis of the bull's eye map (step S104).

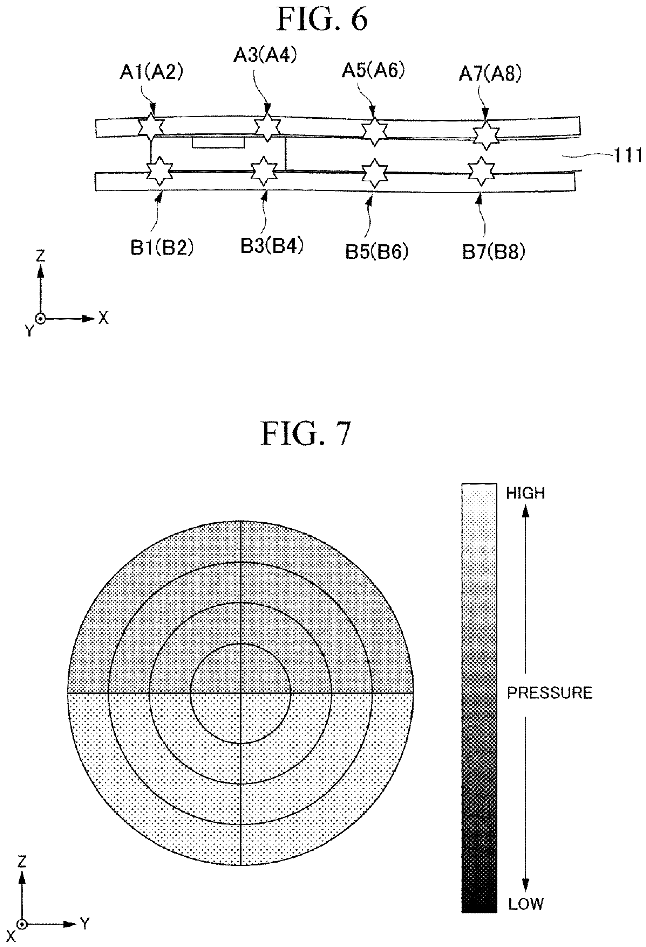

Fig. 6 is a diagram schematically showing a state when the insertion part 111 has been inserted into the body cavity. In the illustrated example, both the front surface and the back surfaces of the insertion part 111 are in contact with the inner wall of the body cavity, and approximately the same pressure is applied to the pressure sensors A and B arranged and disposed at regular intervals in the extending direction (X direction) of the insertion part 111.

Fig. 7 is a diagram showing an example of a bull's eye map in the case of Fig. 6. As illustrated, the entire bull's eye map may be rendered in substantially the same color. In the case of such a bull's eye map, it can be determined that a constant pressure is applied to the front side and the back side of the insertion part 111 and that there is no difference between the pressures on both sides. Therefore, the bending degree estimation function 152 estimates that the ultrasonic probe 110 has not been bent (the bending degree is close to zero). Further, both the pressure on the front side and the pressure on the back side of the insertion part 111 are equal to or less than an upper limit value on the bull's eye map. In this case, the risk estimation function 153 estimates that a degree of risk is low.

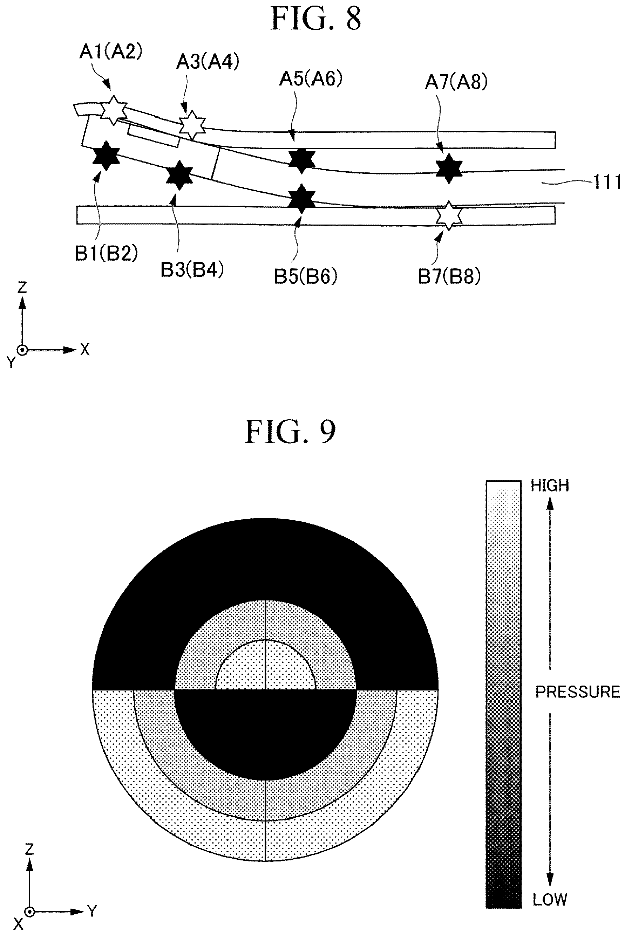

Fig. 8 is a diagram schematically showing a state when the insertion part 111 has been inserted into the body cavity. In the illustrated example, the bending portion 111b is bent as the tip portion 111a of the insertion part 111 comes into contact with the inner wall of the body cavity, and the pressure values of the pressure sensors A1 to A4 provided on the front surface side of the tip portion 111a and the pressure values of the pressure sensors B7 and B8 provided on the back surface side of the guide intermediate portion 111c have increased.

Fig. 9 is a diagram showing an example of a bull's eye map in the case of Fig. 8. As illustrated, the area of the pressure sensors A1 to A4 (area on the center sides of the first and second quadrants) and the area of the pressure sensors B7 and B8 (area outside the third and fourth quadrants) in the bull's eye map are displayed lightly because the pressure values are high, and other areas are displayed darkly because the pressure values are relatively low. In the case of such a bull's eye map, the bending degree estimation function 152 estimates that the ultrasonic probe 110 has been bent at an angle of less than 90 degrees. Moreover, all pressure values are equal to or less than the upper limit value on the bull's eye map. In this case, the risk estimation function 153 estimates that a degree of risk is low.

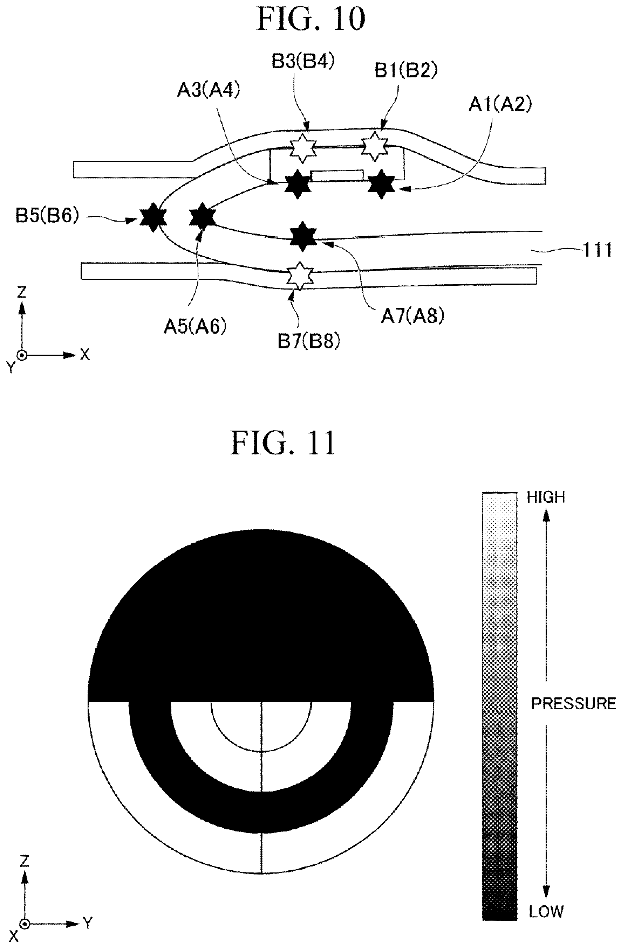

Fig. 10 is a diagram schematically showing a state when the insertion part 111 has been inserted into the body cavity. In the illustrated example, the bending portion 111b is bent, the tip portion 111a of the insertion part 111 is in strong contact with the inner wall of the body cavity, and the pressure values of the pressure sensors B1 to B4 provided on the back surface side of the tip portion 111a and the pressure values of the pressure sensors B7 and B8 provided on the back surface side of the guide intermediate portion 111c have increased.

Fig. 11 is a diagram showing an example of a bull's eye map in the case of Fig. 10. As illustrated, the area of the pressure sensors B1 to B4 (area on the center sides of the third and fourth quadrants) and the area of the pressure sensors B7 and B8 (outermost area of the third and fourth quadrants) in the bull's eye map are displayed lightly because the pressure values are high, and other areas are displayed darkly because the pressure values are relatively low. In the case of such a bull's eye map, the bending degree estimation function 152 estimates that the ultrasonic probe 110 has been bent at an angle of 90 degrees or more. Further, the pressure values of the pressure sensors B1 to B4, B7, and B8 exceed the upper limit value on the bull's eye map. In this case, the risk estimation function 153 estimates that a degree of risk is high.

Note that the risk estimation function 153 may estimate a degree of risk that the ultrasonic probe 110 will damage the inner wall of the body cavity on the basis of the bending degree estimated by the bending degree estimation function 152 in addition to the pressure values of the bull's eye map.

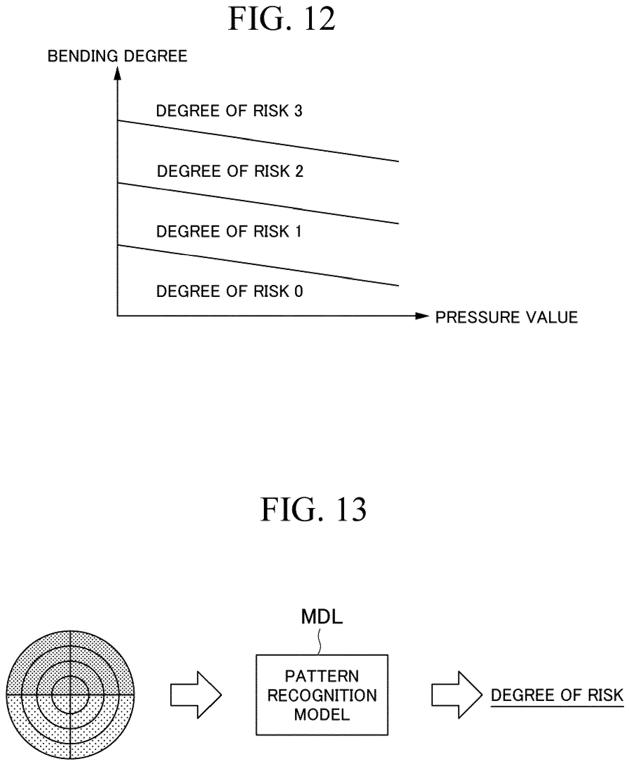

Fig. 12 is a diagram describing a method of estimating a degree of risk using pressure values and a bending degree. For example, the risk estimation function 153 may estimate a degree of risk by using a map in which a degree of risk is associated with a pressure value and a bending degree as shown in Fig. 12. In this map, the higher the pressure value and the higher the bending degree, the higher the degree of risk, and on the other hand, the lower the pressure value and the lower the bending degree, the lower the degree of risk. Such a map may be represented by a table, or may be represented by a function having a pressure value and a bending degree as explanatory variables and a degree of risk as an objective variable.

Further, the risk estimation function 153 may estimate a degree of risk from one image called a bull's eye map using machine learning.

Fig. 13 is a diagram describing a method of estimating a degree of risk using machine learning. For example, it is assumed that there is a certain pattern recognition model MDL. The pattern recognition model MDL is a model supervised-trained to output a degree of risk when a bull's eye map is input, and may be implemented by, for example, a neural network or the like. In other words, the pattern recognition model MDL is a model trained on the basis of training data (a data set in which input data and correct output data are combined) in which output data representing a degree of risk derived from a bull's eye map is associated with input data representing the bull's eye map as a correct label (also called a target). When the bull's eye map is generated, the risk estimation function 153 inputs the bull's eye map to the pattern recognition model MDL. In response to this, the pattern recognition model MDL outputs a plausible degree of risk.

A description of the flowchart of Fig. 4 will now continue. Next, the output control function 156 outputs the bending state (bending degree) estimated by the bending degree estimation function 152 and the degree of risk estimated by the risk estimation function 153 via the output interface 130 (step S106). At this time, the output control function 156 may output the pressure information of each pressure sensor and a bull's eye map which is the pressure distribution via the output interface 130. For example, the output control function 156 may cause the display 130a of the output interface 130 to display the bending state, degree of risk, pressure information, bull's eye map, and the like as images.

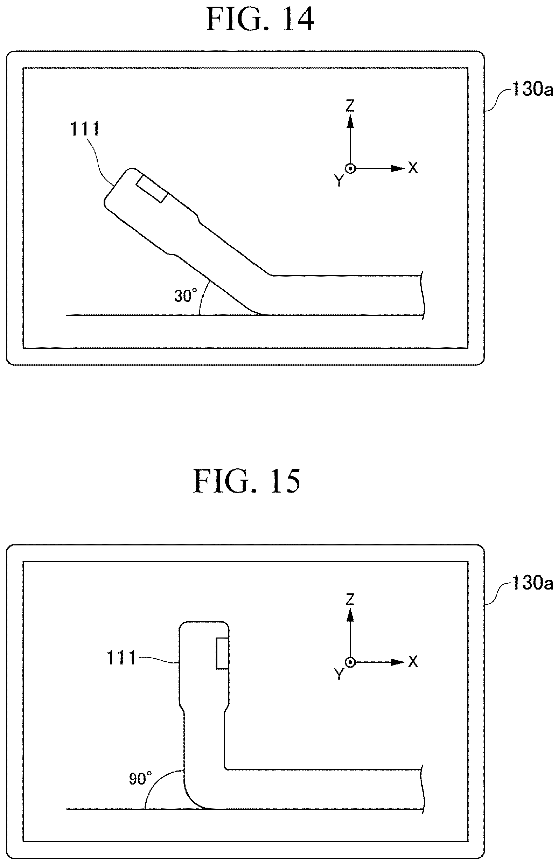

Figs. 14 to 17 are diagrams showing examples of screens of the display 130a. As described above, the bending degree estimation function 152 can roughly estimate a degree of bending from the pressure information of each pressure sensor and the bull's eye map representing the pressure distribution. For example, bending degrees may include almost no bending, less than 90 degrees, approximately 90 degrees, more than 90 degrees, and the like. For example, as shown in Figs. 14 and 15, the output control function 156 may cause the display 130a to display a two-dimensional figure imitating the insertion part 111 in a bending state (bending degree) estimated by the bending degree estimation function 152. In order to estimate a more accurate bending angle, for example, a rotary encoder (not shown) may be installed at a contact portion with a vertical movement control wire operating in association with the vertical bending knob of the operation part 112. It is possible to acquire information about a rotation angle from the rotary encoder at any time, calculate a pulling or extension distance of the wire from the information, and calculate a vertical bending angle according to the distance. At this time, the output control function 156 may display a degree of bending of the insertion part 111 as an angle. At this time, the output control function 156 may display a dialog or the like to call the user's attention as an alert when the degree of risk is higher than a certain level.

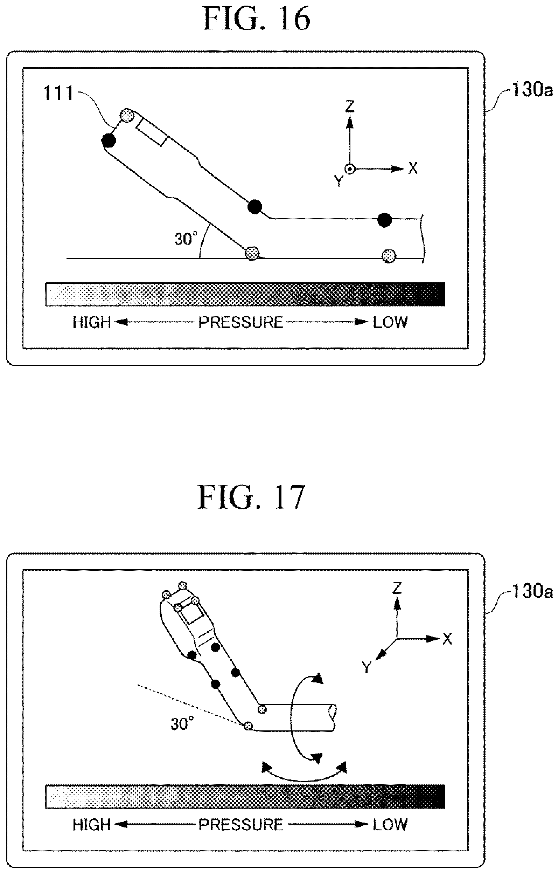

In addition, as shown in Fig. 16, the output control function 156 may superimpose an image that visualizes the position of each pressure sensor and the pressure intensity of each pressure sensor on a two-dimensional figure imitating the insertion part 111. Further, as shown in Fig. 17, the output control function 156 may cause the display 130a to display a three-dimensional figure imitating the insertion part 111 and additionally superimpose an image that visualizes the position of each pressure sensor and the pressure intensity of each pressure sensor on the three-dimensional figure. This three-dimensional figure may be displayed as a polygon, for example, and can be rotated, moved, enlarged, and reduced.

A escription of the flowchart of Fig. 4 will now continue. Next, the probe control function 155 controls the bending degree of the ultrasonic probe 110 on the basis of the bending state of the ultrasonic probe 110 estimated by the bending degree estimation function 152 (step S108). Note that processing of S108 may be omitted. That is, instead of automatically controlling the bending degree of the ultrasonic probe 110 by the ultrasonic diagnostic apparatus 100, the user may be prompted to operate the operation part 112 to manually control the bending degree of the ultrasonic probe 110.

For example, when the bending degree estimation function 152 estimates that the insertion part 111 of the ultrasonic probe 110 has been bent, the probe control function 155 may stretch the insertion part 111 by controlling the actuator of the operation part 112 via the connector part 114. Further, when the bending portion 111b is bent and the tip portion 111a of the insertion part 111 is in strong contact with the inner wall of the body cavity as shown in Fig. 10, a degree of risk that the inner wall of the body cavity will be damaged is high if the insertion part 11 stretches as it is. Accordingly, in such a case, the probe control function 155 prevents the insertion part 111 from stretching by controlling the actuator of the operation part 112 via the connector part 114 and may bend the insertion part 111 if further bending is possible. When the body cavity into which the insertion part 111 is inserted is the esophagus, the output control function 156 may cause the display 130a to display text or an image for prompting the user to insert the insertion part 111 from the esophagus into the stomach while maintaining the current bending state. This allows the insertion part 111 to be stretched in the stomach, which is a wider space than the esophagus, thereby reducing the risk of damage.

Processing of this flowchart ends with such a series of processing. As described above, the processing of this flowchart is repeatedly executed at a predetermined cycle. That is, when pressure information is repeatedly acquired from each pressure sensor by the acquisition function 151 at a predetermined cycle, the risk estimation function 153 repeats generation of a bull's eye map on the basis of the repeatedly acquired pressure information each time pressure information is repeatedly acquired by the acquisition function 151. The bending degree estimation function 152 repeats estimation of a bending state of the ultrasonic probe 110 on the basis of repeatedly generated bull's eye maps each time a bull's eye map is repeatedly generated. Similarly, the risk estimation function 153 repeats estimation of a degree of risk on the basis of repeatedly generated bull's eye maps each time a bull's eye map is repeatedly generated. Then, the output control function 156 repeats output of information such as pressure information, a bending state, and a degree of risk.

According to the first embodiment described above, the acquisition function 151 acquires pressure information from each of the plurality of pressure sensors provided on the insertion part 111 of the ultrasonic probe 110. The risk estimation function 153 generates a bull's eye map, which is a pressure distribution diagram, on the basis of the pressure information of each pressure sensor acquired by the acquisition function 151 and the installation position of each pressure sensor. The bending degree estimation function 152 estimates a bending state of the ultrasonic probe 110 on the basis of the bull's eye map. The output control function 156 causes the output interface 130 to output information such as the pressure information, the bending state, and a degree of risk (that is, information representing a state of the ultrasonic probe 110). As a result, safety and diagnostic efficiency can be improved.

For example, when the bending state of the ultrasonic probe 110 is displayed on the display 130a, the operator of the ultrasonic probe 110 can check whether the ultrasonic probe 110 has been bent or not. As a result, it is possible to reduce the risk of the tip of the ultrasonic probe 110 being caught on the inner wall of the body cavity and damaging the subject when the operator intends to remove the ultrasonic probe 110 in a bent state from the body cavity. That is, the bending state of the ultrasonic probe 110 actually inserted into the body cavity can be estimated from the pressure information of each of the plurality of pressure sensors, and at the same time, it is possible to warn the operator that there is a possibility of damaging the wall surface of the body cavity due to removal or the like, and thus patient safety can be ensured.

In addition, since the operator can check a degree of bending of the ultrasonic probe 110 inserted into the body cavity, the transducer T can be appropriately brought into contact with a target part, thereby improving diagnostic efficiency.

Furthermore, as described above, the technology according to the present embodiment is generally applicable to ultrasonic probes that may be bent during diagnosis in addition to ultrasonic probes for transesophageal use. That is, the technology according to the present embodiment is not limited to transesophageal echocardiography probes and is applicable to general ultrasonic probes inserted into body cavities, such as transvaginal ultrasonic probes and transrectal ultrasonic probes.

### (Second embodiment)

Hereinafter, a second embodiment will be described. The second embodiment differs from the above-described first embodiment in that it is determined whether or not the transducer T is in contact with the inner wall of the body cavity with an optimum pressure. In the following, differences from the first embodiment will be mainly described, and description of common points with the first embodiment will be omitted. In the description of the second embodiment, the same parts as those of the first embodiment are denoted by the same reference numerals.

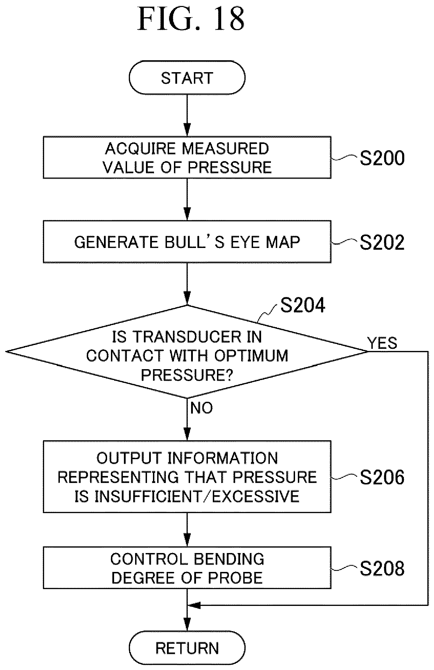

Fig. 18 is a flowchart showing a series of processing of the processing circuitry 150 of the second embodiment. The processing of this flowchart may be repeatedly executed at a predetermined cycle, for example.

First, the acquisition function 151 acquires pressure information from each pressure sensor of the ultrasonic probe 110 in accordance with a predetermined cycle (step S200).

Next, the risk estimation function 153 generates a bull's eye map on the basis of the pressure information acquired from each pressure sensor by the acquisition function 151 and the installation position of each pressure sensor (step S202).

Next, the bending degree estimation function 152 determines whether or not the transducer T of the insertion part 111 is in contact with the inner wall of the body cavity with an optimum pressure on the basis of the bull's eye map (step S204).

For example, the bending degree estimation function 152 may determine that the transducer T is in contact with the inner wall of the body cavity with an optimum pressure if pressure values of the pressure sensors A1 to A4 provided on the same surface as the transducer T, that is, on the front surface side, among the pressure sensors A1 to A4 and B1 to B4 provided on the tip portion 111a, are within a predetermined numerical range (pressure range that can be regarded as optimum) on the bull's eye map, and may determine that the transducer T is not in contact with the inner wall of the body cavity with the optimum pressure if the pressure values of the pressure sensors A1 to A4 do not fall within the predetermined numerical range.

If it is determined that the transducer T is in contact with the inner wall of the body cavity with the optimum pressure, processing of this flowchart may end.

On the other hand, if it is determined that the transducer T is not in contact with the inner wall of the body cavity with the optimum pressure, the output control function 156 outputs information representing that the pressure required to bring the transducer T into contact with the inner wall of the body cavity is insufficient or information representing that the pressure is excessive via the output interface 130 (step S206).

Next, the probe control function 155 controls the bending degree of the ultrasonic probe 110 such that the pressure when the transducer T is brought into contact with the inner wall of the body cavity is optimized (step S208). Note that processing of S108 may be omitted.

According to the second embodiment described above, the bending degree estimation function 152 determines whether or not the transducer T is in contact with the inner wall of the body cavity with an optimum pressure on the basis of a bull's eye map. When it is determined that the transducer T is not in contact with the inner wall of the body cavity with an optimum pressure, the output control function 156 outputs information representing that the pressure is insufficient or excessive via the output interface 130, or the probe control function 155 controls the bending degree of the ultrasonic probe 110 such that the pressure is optimized. As a result, a high-quality ultrasonic image can be generated, and diagnostic efficiency can be further improved.

In general, controlling the bending degree of the ultrasonic probe 110 such that the transducer T is brought into close contact with the inner wall of the body cavity with an appropriate pressure is achieved by the operator checking an image displayed on the display 130a through experience or feeling a subtle resistance of the ultrasonic probe 110 at hand. Therefore, an objective and appropriate amount of bending cannot be ascertained to cause a burden on the inner wall by pressing the probe against the inner wall with more pressure than necessary, or an ultrasonic image with a sufficient signal-to-noise (S/N) ratio cannot be acquired due to pressing with insufficient bending, which may reduce diagnostic efficiency.

On the other hand, in the second embodiment, the operator is informed whether or not the transducer T is in contact with the inner wall of the body cavity with an optimum pressure, and the bending degree of the ultrasonic probe 110 is controlled such that the pressure is optimized (the transducer T is in contact with the inner wall more closely), and thus it is possible to improve diagnostic operation efficiency and enhance the throughput.

### (Other embodiments)

Hereinafter, other embodiments will be described. Although the processing circuitry 150 generates a bull's eye map, which is a pressure distribution diagram, on the basis of pressure information and the installation position of each pressure sensor, estimates a degree of bending of the ultrasonic probe 110, and additionally estimates a degree of risk of damaging the inner wall of the body cavity by the ultrasonic probe 110 in the above-described embodiment, the present invention is not limited thereto. For example, the processing circuitry 150 may estimate a degree of bending or a degree of risk of the ultrasonic probe 110 on the basis of the pressure information and installation position of each pressure sensor without generating a bull's eye map.

While several embodiments of the present invention have been described, these embodiments have been presented by way of example only, and are not intended to limit the scope of the inventions. These embodiments may be embodied in a variety of other forms. Various omissions, substitutions, and combinations may be made without departing from the scope of the inventions as defined by the appended claims.

### EXPLANATION OF REFERENCES

100 Ultrasonic diagnostic apparatus
110 Ultrasonic probe
120 Input interface
130 Output interface
140 Communication interface
150 Processing circuitry
151 Acquisition function
152 Bending degree estimation function
153 Risk estimation Function
154 Image generation function
155 Probe control function
156 Output control function
160 Memory

## Claims

1. An ultrasonic diagnostic apparatus (100) comprising:
an acquisition unit (151) configured to acquire contact information detected by a plurality of contact sensors (A1 - A8, B1 - B8) provided at different positions on an ultrasonic probe (110) configured to be inserted into a body cavity of a subject; and
an output control unit (156) configured to cause an output interface (130) to output information representing a state of the ultrasonic probe (110) in the body cavity on the basis of the contact information and the positions of the contact sensors (A1 - A8, B1 - B8) provided on the ultrasonic probe (110).

2. The ultrasonic diagnostic apparatus according to claim 1, further comprising a generation unit (154) configured to generate a contact information map representing a relationship between the positions of the contact sensors (A1 - A8, B1 - B8) and the contact information,
wherein the output control unit (156) is configured to cause the output interface (130) to output the contact information map as the information representing a state of the ultrasonic probe (110).

3. The ultrasonic diagnostic apparatus according to claim 2, wherein the generation unit (154) is configured to generate, as the contact information map, a bull's eye map in which the positions at which the contact sensors (A1 - A8, B1 - B8) are provided on the ultrasonic probe (110) are represented by distances and directions from a center of a circle and the contact information is represented as a heat map.

4. The ultrasonic diagnostic apparatus according to any of claims 1 to 3, further comprising an estimation unit (152) configured to estimate a degree of bending of the ultrasonic probe (110) in the body cavity on the basis of the positions of the contact sensors (A1 - A8, B1 - B8) and the contact information,
wherein the output control unit (156) is configured to cause the output interface to output information representing the degree of bending as the information representing a state of the ultrasonic probe (110).

5. The ultrasonic diagnostic apparatus according to claim 4, wherein the output control unit (156) is configured to cause the output interface (130) to output a two-dimensional or three-dimensional figure schematically showing the ultrasonic probe (110) bent according to the degree of bending.

6. The ultrasonic diagnostic apparatus according to claim 5, wherein the output control unit (156) is configured to superimpose the positions of the contact sensors (A1 - A8, B1 - B8) and a degree of contact with an inner wall of the body cavity on the figure.

7. The ultrasonic diagnostic apparatus according to claim 1, further comprising an estimation unit (153) configured to estimate a degree of risk of damaging the inner wall of the body cavity on the basis of the positions of the contact sensors (A1 - A8, B1 - B8) and the contact information,
wherein the output control unit (156) is configured to cause the output interface (130) to output information representing the degree of risk as the information representing a state of the ultrasonic probe (110).

8. The ultrasonic diagnostic apparatus according to any preceding claim, further comprising a probe control unit (155) configured to control a degree of bending of the ultrasonic probe (110) on the basis of the positions of the contact sensors (A1 - A8, B1 - B8) and the contact information.

9. The ultrasonic diagnostic apparatus according to any preceding claim, wherein the acquisition unit (151) is configured to input the positions of the contact sensors (A1 - A8, B1 - B8) and the contact information to a model that has undergone supervised learning on the basis of training data, and acquires results output by the model in response to input of the positions of the contact sensors (A1 - A8, B1 - B8) and the contact information, and the output control unit (156) is configured to cause the output interface (130) to output the results output by the model as the information representing a state of the ultrasonic probe (110),
wherein the training data is a data set in which input data indicating the positions of the contact sensors (A1 - A8, B1 - B8) and the contact information is associated with output data indicating a state of the ultrasonic probe (110).

10. The ultrasonic diagnostic apparatus according to any preceding claim, further comprising:
a determination unit configured to determine whether or not a transducer surface of the ultrasonic probe (110) has come into contact with the inner wall of the body cavity with a predetermined force on the basis of the contact information; and
a probe control unit (155) configured to control a degree of bending of the ultrasonic probe (110) such that the transducer surface comes into contact with the inner wall of the body cavity with a predetermined force when the transducer surface has not come into contact with the inner wall of the body cavity with a predetermined force.

11. The ultrasonic diagnostic apparatus according to any one of claims 1 to 10, wherein the acquisition unit (151) is configured to acquire the presence or absence of contact of the contact sensors (A1 - A8, B1 - B8) with the inner wall of the body cavity as the contact information.

12. The ultrasonic diagnostic apparatus according to any one of claims 1 to 10, wherein the acquisition unit (151) is configured to acquire a pressure value detected by the contact sensors (A1 - A8, B1 - B8) in contact with the inner wall of the body cavity as the contact information.

13. An information-processing method, using an ultrasound diagnostic device, comprising:
acquiring detection results of a plurality of contact sensors (A1 - A8, B1 - B8) provided at different positions on an ultrasonic probe (110) inserted into a body cavity of a subject; and
causing an output interface (130) to output information representing a state of the ultrasonic probe (110) in the body cavity on the basis of the detection results of the contact sensors (A1 - A8, B1 - B8) and the positions at which the contact sensors (A1 - A8, B1 - B8) are provided on the ultrasonic probe (110).

14. A storage medium storing a program causing a computer to execute:
acquiring detection results of a plurality of contact sensors (A1 - A8, B1 - B8) provided at different positions on an ultrasonic probe (110) inserted into a body cavity of a subject; and
causing an output interface (130) to output information representing a state of the ultrasonic probe (110) in the body cavity on the basis of the detection results of the contact sensors (A1 - A8, B1 - B8) and the positions at which the contact sensors (A1 - A8, B1 - B8) are provided on the ultrasonic probe (110).
